(19) Europäisches Patentamt
European Patent Office
Office européen des brevets

(11) EP 4 249 910 A1

(12) EUROPEAN PATENT APPLICATION
published in accordance with Art. 153(4) EPC

(43) Date of publication:
27.09.2023 Bulletin 2023/39

(51) International Patent Classification (IPC):
G01N 33/48 (2006.01)    C12Q 1/06 (2006.01)

(21) Application number: 21894610.1

(52) Cooperative Patent Classification (CPC):
C12Q 1/06; G01N 33/48

(22) Date of filing: 16.11.2021

(86) International application number:
PCT/JP2021/041981

(87) International publication number:
WO 2022/107736 (27.05.2022 Gazette 2022/21)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 17.11.2020 JP 2020191078

(71) Applicant: Hirotsu Bio Science Inc.
Tokyo 102-0094 (JP)

(72) Inventor: HIROTSU, Takaaki
Tokyo 102-0094 (JP)

(74) Representative: Plasseraud IP
66, rue de la Chaussée d'Antin
75440 Paris Cedex 09 (FR)

(54) BLOOD SAMPLE TESTING METHOD USING TAXIS BEHAVIOR OF NEMATODE

(57) The present invention provides a method for analyzing a blood sample. According to the present invention, there is provided, for example, a method for testing or analyzing a blood sample from a subject having cancer or suspected of having cancer, including evaluating taxis behavior of nematodes to the blood sample. According to the present invention, there is provided, for example, a method for evaluating whether a subject has cancer or not by the above method.

FIG. 4

**Description**

Technical Field

The present invention relates to a method for testing blood samples based on taxis behavior of nematodes.

Background Art

Cancer screening tests using characteristic of nematodes: being attracted by cancer-specific smell generated in biological substances or treated materials thereof have been developed. Patent Literature 1 discloses a method for determining whether a subject from which a urine sample is derived is likely to have cancer based on difference in response of nematodes to the urine sample.

Citation List

Patent Literature

Patent Literature 1: WO2015/088039

Summary of Invention

The present invention provides a method for testing blood samples based on taxis behavior of nematodes.
The present inventors have found that blood samples can be tested or analyzed based on taxis behavior of nematodes. They found that nematodes can exhibit attraction behavior to blood samples derived from cancer patients. They have also found that nematodes can exhibit avoidance behavior to blood samples derived from non-cancer subjects (subjects having no cancer). They have further found that taxis behavioral response of nematodes can be improved by using blood samples reduced in protein content by deproteinization. The present invention has been attained based on these findings.
According to the present invention, there are provided the following inventions.

[1] A method for testing or analyzing a blood sample from a subject having cancer or suspected of having cancer, comprising evaluating taxis behavior of nematodes to the blood sample.
[2] The method according to the above [1], wherein the blood sample is a serum sample or a plasma sample.
[3] The method according to the above [1] or [2], wherein the blood sample is a serum sample or a plasma sample having a reduced protein content.
[4] The method according to any one of the above [1] to [3], wherein the blood sample is a blood sample having a protein content by deproteinization.
[5] The method according to the above [4], wherein the deproteinization is deproteinization with a solution containing at least one selected from the group consisting of methanol, formic acid and trichloroacetic acid, or deproteinization by ultrafiltration.
[6] The method according to any one of the above [1] to [5], wherein the cancer is solid cancer.
[7] The method according to the above [6], wherein the cancer is gastrointestinal cancer.

According to the present invention, blood samples can be analyzed.

Brief Description of Drawings

[Figure 1] Figure 1 shows evaluation results of taxis behaviors of nematodes to urine samples obtained from 2 cancer patients and 4 cancer-negative human (non-cancer subjects).
[Figure 2] Figure 2 shows evaluation results of taxis behaviors of nematodes to plasma samples obtained from 2 cancer patients and 2 non-cancer subjects.
[Figure 3] Figure 3 shows evaluation results of taxis behaviors of nematodes to serum samples obtained from 2 cancer patients and 2 non-cancer subjects.
[Figure 4] Figure 4 shows evaluation results of taxis behaviors of nematodes to deproteinized plasma samples obtained by subjecting plasma samples obtained from of 2 cancer patients and 2 non-cancer subjects to deproteinization.

[Figure 5] Figure 5 shows evaluation results of taxis behaviors of nematodes to deproteinized serum samples obtained by subjecting serum samples obtained from of 2 cancer patients and 2 non-cancer subjects to deproteinization.

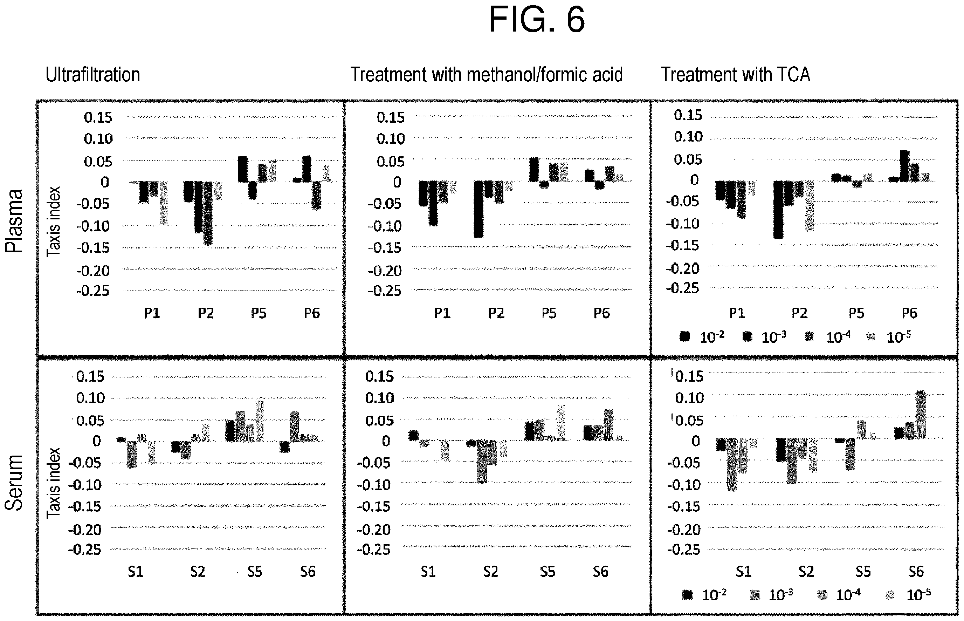

[Figure 6] Figure 6 shows a summary of Figure 4 and Figure 5.

Detailed Description of the Invention

As used herein, a "subject" refers to a mammal, for example, a human. Also, examples of the "subject" as used herein include a healthy subject, a subject suspected of having cancer and a subject suffering from cancer. Examples of the "subject suspected of having cancer" as used herein include a subject definitely suspected of having cancer and a subject who has never been specifically suspected of having cancer.

As used herein, "cancer" refers to a malignant tumor. Cancers can be roughly classified into a group of hematopoietic tumors and a group of solid tumors. Examples of the hematopoietic tumors include leukemia, malignant lymphoma and myeloma. Examples of the solid tumors include solid cancers such as gastrointestinal cancers including stomach cancer, esophageal cancer, colon cancer, colorectal cancer, gallbladder cancer and pancreatic cancer, lung cancer, breast cancer, liver cancer, uterine cancer, ovarian cancer, head and neck cancer and tongue cancer; sarcomas such as osteosarcoma, chondrosarcoma, rhabdomyosarcoma, leiomyosarcoma, fibrosarcoma, liposarcoma and angiosarcoma; sarcomas such as chondrosarcoma, Ewing's sarcoma, malignant hemangioendothelioma, malignant schwannoma, osteosarcoma and soft tissue sarcoma, and blastomas such as hepatoblastoma, medulloblastoma, nephroblastoma, neuroblastoma, pancreatoblastoma, pleuropulmonary blastoma and retinoblastoma.

As used herein, the phrase "detecting cancer" can be rephrased by "detecting cancer cells", "identifying cancer", "determining cancer", "a method for assisting diagnosis of cancer" or "a method of acquiring preliminary information for diagnosis of cancer". The method of the present invention is an industrially applicable method. In an embodiment, the method of the present invention does not correspond to a method carried out by doctors. In an embodiment, the method of the present invention does not involve medical practice.

As used herein, "nematodes" refer to *Caenorhabditis elegans.* Nematode strains, which have been isolated from various environments, are registered, as public data, at the Caenorhabditis Genetics Center (CGC) of Biological Science in the University of Minnesota, USA and can be handed over. For those skilled in the art, almost all strains so far known are available from CGC. A hermaphrodite is preferably used because it is reproducible by self-fertilization.

As used herein, "taxis behavior" refers to attraction behavior or avoidance behavior. The attraction behavior refers to a behavior of decreasing a physical distance from a substance, whereas the avoidance behavior refers to a behavior of increasing the physical distance from a substance. The substance inducing attraction behavior refers to an attractant, whereas the substance inducing avoidance behavior refers to a repellent. Nematodes have a characteristic of being attracted by an attractant and moving away from a repellent due to olfactory perception. The behavior being attracted by an attractant is referred to as attraction behavior (herein sometimes referred as to "positive"); whereas the behavior moving away from a repellent is referred to as avoidance behavior (herein sometimes referred as to "negative"). The attraction behavior and avoidance behavior are collectively referred to as taxis behavior.

As used herein, a "wild strain" refers to the wild nematode strain. A typical wild strain is, for example, N2 Bristol strain.

As used herein, a "blood sample" refers to a blood-containing sample obtained from a subject. Examples of the blood sample include a serum sample and a plasma sample. As used herein, a "test sample" refers to a sample to be examined.

According to the present invention, there is provided a method for testing (or analyzing) a biological sample (especially, blood sample) from a subject having cancer or suspected of having cancer. Also, according to the present invention, the method of the present invention can be a method for detecting (or predicting, diagnosing or acquiring basic information of therapeutic effect on) cancer in a subject having cancer or suspected of having cancer, including evaluating taxis behavior of nematodes to a urine sample from the subject. The method of the present invention can be an industrially applicable invention. The method of the present invention can be a non-medical method.

As the nematode, a wild type nematode (e.g., N2 Bristol strain) can be used. The nematode strain, since it exhibits attraction behavior to urine of cancer patients, can be used in the present invention.

A blood sample can be obtained from a subject. A blood sample, if it is evaluated by the method of the present invention, is diluted with an aqueous solvent such as water and used as a diluted sample. The dilution factor herein may be, for example, 5 times or more, 10 times or more, 20 times or more, 30 times or more, 40 times or more, 50 times or more, 60 times or more, 70 times or more, 80 times or more, 90 times or more, 100 times or more, 200 times or more, 300 times or more, 400 times or more, 500 times or more, 600 times or more, 700 times or more, 800 times or more, 900 times or more, 1,000 times or more, 2,000 times or more, 3,000 times or more, 4,000 times or more, 5,000 times or more, 6,000 times or more, 7,000 times or more, 8,000 times or more, 9,000 times or more, or 10,000 times or more. The dilution factor may be also 100 times or less, 200 times or less, 300 times or less, 400 times or less, 500 times or less, 600 times or less, 700 times or less, 800 times or less, 900 times or less, 1,000 times or less, 2,000 times or less, 3,000 times or less, 4,000 times or less, 5,000 times or less, 6,000 times or less, 7,000 times or less, 8,000 times or less, 9,000 times or less, or 10,000 times or less. In an aspect, a blood sample can be diluted 5 times to 100,000 times, for example, 10 times to 10,000 times, 50 times to 2,000 times, 50 times to 500 times, or 500 times to 5,000 times and used as a diluted sample.

A urine sample can be obtained from a subject. A urine sample, if it is evaluated by the method of the present invention, may be diluted with a solvent such as water. If the sample is diluted, the dilution factor herein may be, for example, 5 times to 20000 times, 10 times to 10000 times, 10 times to 5000 times, 10 times to 2000 times, or 100 times to 1000 times. Nematodes exhibit taxis behavior to diluted urine samples. In an aspect of the present invention, the dilution factor of urine can be, for example, 100 times or 1000 times. Alternatively, two or more dilution factors including 100 times and 1000 times may be employed. For example, the dilution factor of a dog urine sample may be 10 times to 2000 times, for example, 100 times to 1000 times. For example, the dilution factor of a cat urine sample may be 5 times to 20000 times or 10 times to 10000 times, for example, 500 times to 5000 times. For example, the dilution factor of a mouse urine sample may be 5 times to 20000 times or 10 times to 10000 times, for example, 500 times to 5000 times.

Those skilled in the art can appropriately determine the dilution factor of a sample by preparing serial dilutions (e.g., 2 times to 10 times serial dilution) of test samples (e.g., blood sample) obtained from cancer patients and non-cancer subjects, evaluating taxis behaviors of nematodes to the serially diluted samples of urine obtained from cancer patients and non-cancer patients and determining the dilution factor of the sample at which responses of nematodes are satisfactorily distinguishable. The dilution factor can be, for example, 100 times to 20,000 times, 500 times to 10,000 times or 1,000 times to 10,000 times. To improve accuracy of an examination, examination or analysis can be carried out after a dilution factor, at which excellent discrimination ability is shown, is determined by appropriately conducting studies on dilution concentration. Analysis can be made using a plurality of dilution factors.

Serum or plasma can be obtained from a sample (e.g., blood sample) by a routine method. The serum refers to a liquid component obtained when blood is coagulated. The plasma is a component of blood in which cells are absent. More specifically, the plasma can be the supernatant obtained by centrifugation of blood.

A blood sample may be reduced in protein content by deproteinization and then subjected to analysis of the present invention. Accordingly, the blood sample of the present invention may be a blood sample reduced in protein content. In an embodiment of the present invention, a blood sample may be plasma reduced in protein content. In an embodiment of the present invention, a blood sample may be serum reduced in protein content. The protein content reduced refers to the content of proteins lower than that usually contained in a blood sample. Blood samples reduced in protein content (e.g., serum and plasma) can be obtained by subjecting, for example, blood samples (e.g., serum and plasma) to deproteinization. Although the deproteinization is not particularly limited, a routine method applicable by those skilled in the art can be used. Deproteinization is a method of removing proteins from a sample, for example, by adding at least one chemical agent (e.g., protein denaturant) selected from the group consisting of acids (such as formic acid, perchloric acid, trichloroacetic acid and metaphosphoric acid) and organic solvents (such as acetone, acetonitrile, methanol and ethanol) to denature and insolubilize proteins. Alternatively, deproteinization may be carried out by removing proteins from a sample by a physical means such as ultrafiltration, ultrafiltration using, e.g., a membrane filter (e.g., centrifugal filtration device), dialysis and ultracentrifugation. The resultant blood sample is diluted with a solvent such as water and then subjected to analysis of the present invention. More specifically, blood samples (e.g., plasma and serum) may be diluted blood samples reduced in protein content.

Taxis behavior of nematodes can be evaluated by placing a sample (e.g., urine sample or blood sample) and nematodes in a predetermined distance (e.g., about 0 cm to about 5 cm, for example, distance of 1 cm to 5 cm) and observing whether the nematodes exhibit attraction behavior or avoidance behavior to the sample (e.g., urine sample or blood sample). Taxis behavior can be observed on, for example, a solid medium such as an agar medium.

Method for analyzing test sample by nematodes

A method for analyzing a test sample by nematodes can be carried out by placing a test sample (e.g., urine sample or blood sample) obtained from a subject and nematodes in a predetermined distance and observing whether the nematodes exhibit attraction behavior or avoidance behavior to the sample. If attraction behavior is exhibited, it can be evaluated that the subject suffers from cancer or is likely to suffer from cancer. If avoidance behavior is exhibited, it can be evaluated that the subject has no cancer or is likely to have no cancer. Examples of the analysis method using human specimens are as disclosed in WO2015/088039, which is incorporated herein in its entirety by reference.

To describe more specifically, a method for analyzing a test sample (e.g., urine sample or blood sample) by nematodes may include, for example, placing a test sample (e.g., urine sample or blood sample) obtained from a subject in a petri dish (a petri dish containing e.g., solid medium), placing a test substance and nematodes in a predetermined distance in the petri dish having the test sample therein, and thereafter, allowing the nematodes to move.

A method for analyzing a sample (e.g., urine sample or blood sample) by nematodes may include determining that a subject suffers from cancer or is likely to suffer from cancer if nematodes exhibit, for example, attraction behavior to the test sample. A method for analyzing a sample (e.g., urine sample or blood sample) by nematodes may indicate that a subject from which the sample is derived is likely to suffer from cancer if nematodes exhibit, for example, attraction behavior to the test sample.

Taxis behavior of nematodes can be evaluated based on the difference or ratio in number of nematodes moving toward a test sample and nematodes moving away from the test sample. When evaluation is carried out based on the difference in number of nematodes, if the difference is represented by a positive value, it can be evaluated that the test sample as a whole induces attraction behavior and is derived from a cancer patient; and/or if the difference is represented by a negative value, it can be evaluated that the test sample as a whole induces avoidance behavior and is derived from a subject having no cancer. When evaluation is carried out based on the ratio in number of nematodes, if the ratio is 1 or more, it can be evaluated that the test sample induces attraction behavior and is derived from a cancer subject; and/or if the ratio is less than 1, it can be evaluated that the test sample induces avoidance behavior and is derived from a subject having no cancer. Also, taxis behavior of nematodes can be evaluated based on, for example, the following taxis behavior index.

[Expression 1]

$$\text{(Taxis behavior index)} = \frac{A - B}{A + B}$$

wherein A represents the number of nematodes exhibiting attraction behavior to a test sample and B represents the number of nematodes exhibiting avoidance behavior to the test sample.

If the taxis behavior index represents a positive value, it can be evaluated that the test sample as a whole induces attraction behavior and is derived from a subject having cancer; and if the taxis behavior index represents a negative value, it can be evaluated that the test sample as a whole induces avoidance behavior and is derived from a subject having no cancer.

The closer the taxis behavior index to 1, the larger the ratio of nematodes exhibiting attraction behavior; the closer the taxis behavior index to -1, the larger the ratio of nematodes exhibiting avoidance behavior; and the closer the taxis behavior index to 0, the larger the ratio of nematodes exhibit neither attraction behavior nor avoidance behavior. The larger the absolute value of taxis behavior index, the more definite the behavioral evaluation results. If the number or ratio of nematodes that exhibited taxis behavior is almost equal to that exhibited avoidance behavior, the test sample may be determined as positive (the positive sample may be subjected further to a detailed examination) or the sample may be eliminated from the subjects to be evaluated. In the present invention, individuals from which positive test samples are derived can be subjected further to a detailed examination to determine whether they have cancer or not. When 2 or more test samples different in dilution factor are examined, if any one of the samples (different in dilution factor) provides a positive result, it may be determined that the subject has cancer, in view of sensitivity improvement; or if all of the samples provide a positive result, it may be determined that the subject has cancer, in view of specificity improvement.

The method of the present invention may include evaluating a urine sample obtained from a subject having cancer or suspected of having cancer by the evaluation system for taxis behavior of nematodes, and then evaluating a blood sample obtained from the subject by the evaluation system for taxis behavior of nematodes.

A subject evaluated as having cancer or being likely to have cancer by the method of the present invention may receive definitive diagnosis by a doctor whether or not the subject has cancer or not. A subject evaluated as having cancer or being likely to have cancer by the method of the present invention may further receive a detailed examination for definitive diagnosis. A subject evaluated as having cancer or being likely to have cancer by the method of the present invention can thereafter receive a cancer therapy (e.g., chemotherapy, radiotherapy, surgical resection and combination therapy of these).

According to the present invention, there is provided a composition or kit containing nematodes or eggs thereof for use in the analysis method of the present invention, a method for detecting cancer, a method for detecting cancer cells, a method for identifying cancer, a method for determining cancer, a method for assisting diagnosis of cancer, and a method for acquiring preliminary information for cancer diagnosis. The kit may contain, in addition of nematodes or eggs thereof, an evaluation system (e.g., dish, agar and operating instructions) for nematodes behavior and a part thereof.

According to the present invention, there is provided use of nematodes in the manufacture of a composition or kit for use in the analysis method of the present invention, a method for detecting cancer, a method for detecting cancer cells, a method for identifying cancer, a method for determining cancer, a method for assisting diagnosis of cancer or a method for acquiring preliminary information for cancer diagnosis.

Examples

Example 1: Analysis for chemotactic behavior of nematodes to urine sample and blood sample

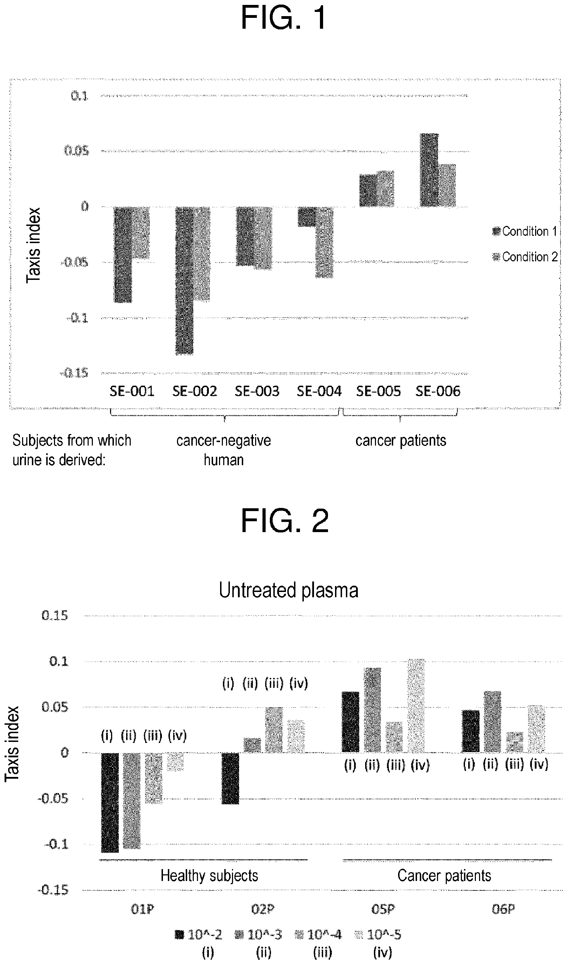

In this example, based on the procedure "(3) detection using olfactory perception of nematodes" disclosed in WO2015/088039, urine samples were taken from 2 human patients having gastric cancer and 4 cancer-negative human subjects, diluted 10 times (Condition 1) and 100 times (Condition 2) and subjected to analysis. As a result, as shown in Figure 1, nematodes exhibited attraction behavior to urine taken from 2 gastric cancer patients and avoidance behavior to urine taken from the cancer-negative human subjects.

Next, blood samples were taken from the 2 gastric cancer patients (SE-005 and SE-006) and 2 subjects (SE-001 and SE-002) selected from the 4 cancer-negative human and subjected to a routine method to obtain serum and plasma. Serum and plasma were not subjected to any treatment or subjected to a treatment with an organic solvent (methanol: formic acid = 1000: 1), a treatment with an acid (10% trichloroacetic acid) or a treatment by ultrafiltration (using Amicon Ultra 3K in accordance with the manufacturer's manual). These samples each were diluted with water. Thereafter, taxis behavior of nematodes to each of the diluted samples was evaluated in accordance with the above procedure.

(1) Taxis of nematodes to untreated plasma/serum

<Method>

Taxis of nematodes to the untreated plasma and serum derived from subjects SE-001, SE-002, SE-005 and SE-006 used in Example 1 were further checked. The untreated plasma and serum were each diluted with water. Four dilution factors: $10^{-2}$, $10^{-3}$, $10^{-4}$ and $10^{-5}$ were employed. The dilution factor of the samples to which attraction behavior in a cancer patient was observed, was confirmed.

<Results>

With respect to the plasma ($10^{-2}$ dilution), as shown in Figure 2, avoidance behavior was exhibited to healthy subject specimens, whereas attraction behavior was exhibited to cancer patient, similarly to the urine specimens of Example 1. With respect to the plasma of healthy subjects, as the dilution factor increases, behavioral change toward attraction behavior was observed. In contrast, with respect to the plasma of cancer patients, attraction behavior was observed at any one of dilution factors.

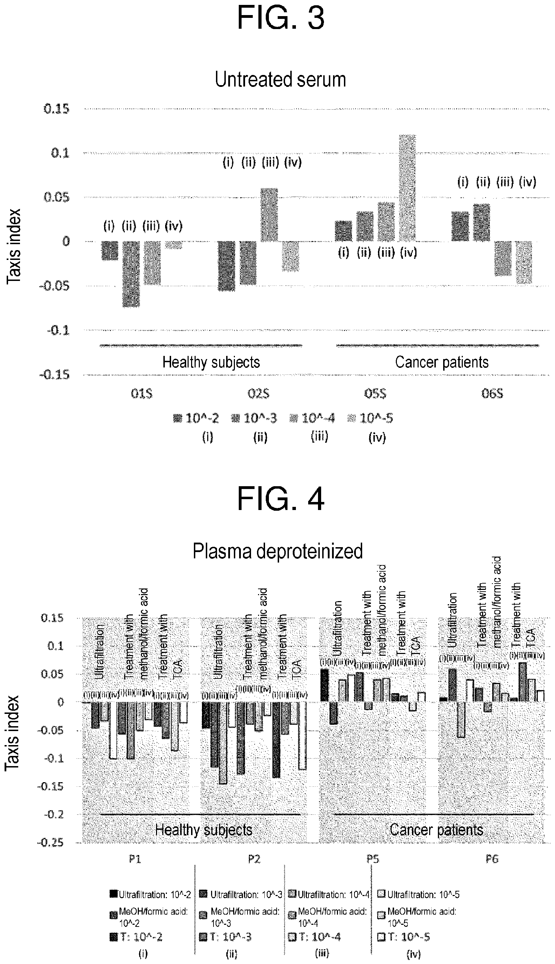

With respect to the serum ($10^{-2}$ or $10^{-3}$ dilution factor), as shown in Figure 3, avoidance behavior was exhibited to healthy subject specimens, whereas attraction behavior was exhibited to cancer patient. Even if the dilution factor was increased, the same tendency as above was not observed.

(2) Taxis of nematodes to plasma/serum deproteinized

<Method>

The plasma and serum each were subjected to deproteinization, which was carried out by any one of three methods: ultrafiltration (Amicon Ultra 3K), methanol/formic acid (1000:1) treatment and 10% trichloroacetic acid (TCA) treatment, to obtain blood samples. The blood samples were further diluted with water. The dilution factors of the blood samples were $10^{-2}$, $10^{-3}$, $10^{-4}$ and $10^{-5}$. Thereafter, taxis of nematodes to diluted samples were evaluated. Note that in the cases of deproteinization with the methanol/formic acid treatment, the supernatants of the samples were diluted double with water and used as blood samples.

<Results>

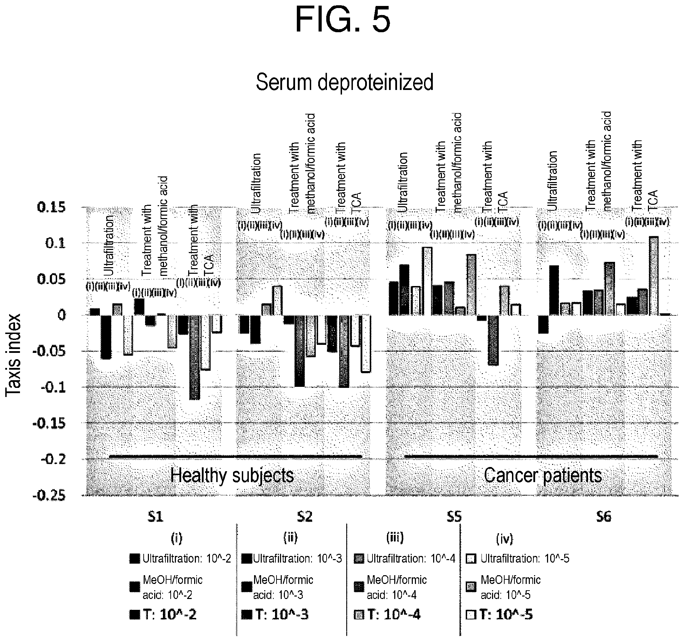

As shown in Figures 4 and 5, in the examination using plasma and serum, it was found that response of nematodes, more specifically, the ability of nematodes, to discriminate between cancer subjects and healthy subjects, tends to increase even if any one of the deproteinization treatments was used. In the examination using plasma, the ability of nematodes to discriminate them is higher than in the examination using serum.

In the examination using serum, it was found that the deproteinization treatment with methanol/formic acid or TCA is more effective and excellent in the discrimination ability.

**Claims**

1. A method for testing or analyzing a blood sample from a subject having cancer or suspected of having cancer, comprising evaluating taxis behavior of nematodes to the blood sample.

2. The method according to claim 1, wherein the blood sample is a serum sample or a plasma sample.

3. The method according to claim 1 or 2, wherein the blood sample is a serum sample or a plasma sample having a reduced protein content.

4. The method according to any one of claims 1 to 3, wherein the blood sample is a blood sample having a reduced protein content by deproteinization.

5. The method according to claim 4, wherein the deproteinization is deproteinization with a solution containing at least one selected from the group consisting of methanol, formic acid and trichloroacetic acid, or deproteinization by ultrafiltration.

6. The method according to any one of claims 1 to 5, wherein the cancer is solid cancer.

7. The method according to claim 6, wherein the cancer is gastrointestinal cancer.

# FIG. 1

Subjects from which          cancer-negative          cancer patients
urine is derived:                    human

# FIG. 2

## FIG. 3

### Untreated serum

## FIG. 4

### Plasma deproteinized

# FIG. 5

Serum deproteinized

FIG. 6

Ultrafiltration | Treatment with methanol/formic acid | Treatment with TCA

Plasma — Taxis index

Serum — Taxis index

P1  P2  P5  P6

S1  S2  S5  S6

■ 10⁻² ■ 10⁻³ ■ 10⁻⁴ ■ 10⁻⁵

<div align="center">

**INTERNATIONAL SEARCH REPORT**

</div>

| International application No. |
|---|
| **PCT/JP2021/041981** |

**A.     CLASSIFICATION OF SUBJECT MATTER**

*G01N 33/48*(2006.01)i; *C12Q 1/06*(2006.01)i
FI:    G01N33/48 N; C12Q1/06; G01N33/48 B

According to International Patent Classification (IPC) or to both national classification and IPC

**B.     FIELDS SEARCHED**

Minimum documentation searched (classification system followed by classification symbols)

G01N33/48; C12Q1/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C.     DOCUMENTS CONSIDERED TO BE RELEVANT**

| Category* | Citation of document, with indication, where appropriate, of the relevant passages | Relevant to claim No. |
|---|---|---|
| X | HIROTSU, Takaaki et al. A Highly Accurate Inclusive Cancer Screening Test Using Caenorhabtis elegans Scent Detection. PLoS ONE. 11 March 2015, vol. 10, no. 3, e0118699 Results and Discussion | 1-2, 6-7 |
| X | JP 2018-44781 A (HIROTSU BIO SCIENCE INC) 22 March 2018 (2018-03-22) claims, paragraphs [0014], [0076] | 1-2, 6-7 |
| X | WO 2017/081750 A1 (HITACHI LTD) 18 May 2017 (2017-05-18) claims, paragraph [0070] | 1-2 |
| X | CN 108424951 A (CHONGQING PUROTON BIOLOGICAL INFORMATION TECHNOLOGY CO., LTD.) 21 August 2018 (2018-08-21) claims, paragraph [0016] | 1-2 |
| A | US 2017/0227491 A1 (THE TRUSTEES OF THE UNIVERSITY OF PENNSYLVANIA) 10 August 2017 (2017-08-10) entire text, all drawings | 1-7 |

☐ Further documents are listed in the continuation of Box C.          ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **06 January 2022** | **25 January 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2021/041981**

| Patent document cited in search report | | | Publication date (day/month/year) | Patent family member(s) | | | Publication date (day/month/year) |
|---|---|---|---|---|---|---|---|
| JP | 2018-44781 | A | 22 March 2018 | US claims, paragraphs [0053], [0118] WO TW AU | 2019/0369084 2018/047959 201812627 2017323139 | A1 A1 A A1 | |
| WO | 2017/081750 | A1 | 18 May 2017 | (Family: none) | | | |
| CN | 108424951 | A | 21 August 2018 | (Family: none) | | | |
| US | 2017/0227491 | A1 | 10 August 2017 | WO whole document | 2016/036950 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015088039 A